**Europäisches Patentamt**

(19) **European Patent Office**

**Office européen des brevets**

(11) Publication number : **0 294 442 B1**

# (12) EUROPEAN PATENT SPECIFICATION

(45) Date of publication of patent specification :
**19.08.92 Bulletin 92/34**

(51) Int. Cl.⁵ : **A61B 17/00, A61B 17/11, A61M 29/02**

(21) Application number : **88900479.2**

(22) Date of filing : **11.12.87**

(86) International application number :
**PCT/SE87/00596**

(87) International publication number :
**WO 88/04539 30.06.88 Gazette 88/14**

(54) **INSTRUMENT FOR PREVENTION OF CONSTRICTIONS IN THE JOINT AREA AT THE APPLIANCE OF BRANCHING ON VESSEL.**

(30) Priority : **18.12.86 SE 8605455**

(43) Date of publication of application :
**14.12.88 Bulletin 88/50**

(45) Publication of the grant of the patent :
**19.08.92 Bulletin 92/34**

(84) Designated Contracting States :
**AT BE CH DE FR GB IT LI LU NL SE**

(56) References cited :
**WO-A-82/01644**
**GB-A- 1 538 737**
**US-A- 3 438 375**
**US-A- 4 523 592**

(73) Proprietor : **UNILINK INC.**
**P.O. Box 720**
**Centreville, VA 22020 (US)**

(72) Inventor : **BERGGREN, Anders**
**Bergdalsgatan 6**
**S-582 45 Linköping (SE)**
Inventor : **RHOMAN, Hakan**
**Vitsippestigen 4**
**S-590 20 Mantorp (SE)**
Inventor : **RAGNARSSON, Rafn**
**Kvinnebyvägen 131**
**S-582 60 Linköping (SE)**

(74) Representative : **Onn, Thorsten et al**
**AB Stockholms Patentbyra Zacco & Bruhn**
**P.O. Box 3129**
**S-103 62 Stockholm (SE)**

EP 0 294 442 B1

## Description

In the vessel surgery it has become increasingly useful with by-passes, i.e. for instance constricted vessels are replaced by healthier ones that are coupled in parallel or that vessels are connected at other places than originally. Up till now one has used the technique of sewing the vessels together by means of sutures. It has however been found that anastomosis rings can be used to make anastomosis "end to side". When this is done the vessel that is to be branched off is punctuated by a small cut that is then flared and the vessel is placed over the pins of the anastomosis ring. In this way it is possible to make the branching or by-passing considerably faster than with a conventional sewing technique. In this way the resources are better used and the risks for the patient are diminished. Regretfully this way of conducting an end to side operation is not entirely without draw-backs. It has namely been found that the applying of the anastomosis ring so to say steals material from the vessel to which the branching is coupled and results in a constriction which definitely is undesirable since the by-pass is applied in order to improve the flow. In view of this problem the invention has as it's object to provide an instrument eliminating this draw-back.

In accordance with the invention these problems are solved by means of a T-shaped instrument where the upper part of the T is constituted by an elastically expandable part that in the most simple case can be constituted by a piece of a thin-walled hose, the vertical part of the T being constituting by another unyielding hose connectable with a pressure source. This pressure source can for instance be a common injection syringe. By providing a medium under pressure to the elastic upper part of the T this can be expanded and by evacuating this part it can also be made very thin and easy to handle. At use the upper parts of the T are each inserted in different directions in the vessel through the little cut where the branching is to be placed. When the parts of the T are well inserted into the vessel they are expanded and the vessel is expanded to about twice of it's original diametre. The instrument is then evacuated and removed from the vessel and an anastomosis ring and the additional vessel end can be connected. Since the vessel is not entirely elastic it will retain its expansion for sufficient time to secure that no constriction occure despite the use of an anastomosis ring for the connection. Tests have shown that no tendency whatever prevail for a rupture or a tearing of the cut in the vessel during the expansion of this. The reason for this may be the very unifrome stress that the vessel is subjected to in comparison with what would be the case if one tries to expand the vessel by means of pincettes or the like.

A T-shaped instrument for use during an anastomosis operation is known from GB-A- 1 538 737 which discloses an instrument for stopping the flow of blood during an anastomosis operation.

At each end of the uppert part of the T inflatable balloons are formed, whereas the T's vertical part is constituted by an unyielding pipe or hose. The upper part of the T constitutes a closed space but for a connection with the vertical hose or pipe.

The invention is below to be described in connection with the drawing, where fig. 1 shows an embodiment in accordance with the invention seen from the side at rest and fig. 2 the same instrument seen from the side and fig. 3 shows the instrument in it's so to say working state.

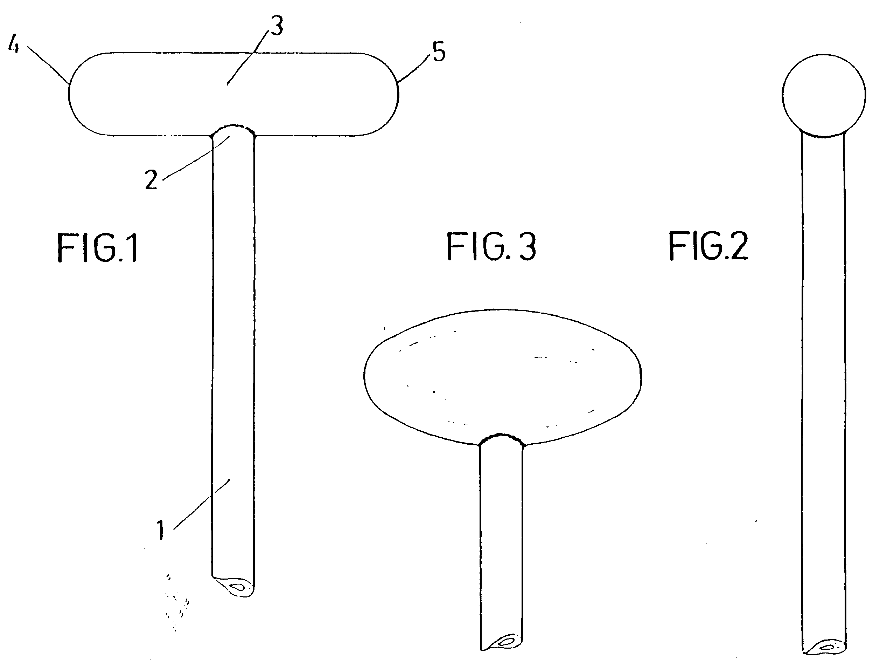

The instrument shown in fig. 1 is constituted by a thick-walled connection hose 1 of plastic, which has been welded to a side-opening 2 in a thin-walled elastic plastic hose 3. This hose 3 is furthermore at its ends 4 and 5 welded together. The hose 1 is very thin and has preferably such a diameter that it fits on the tip of an injection syringe. In fig. 3 finally the instrument has been subjected to pressure resulting in that the thin-walled upper part of the instrument has been expanded.

On the drawing the instrument is of course shown enlarged, in reality the diameter of the connection hose 1 is about 1 mm whereas "the balloonpart" has a diameter of about 2 mm and the length of this upper balloonpart is about 8 mm in unaffected state. Finally it should be mentioned that the instrument is very hygenic and economic to produce.

## Claims

1. Instrument for prevention of constrictions in the joint area at the appliance of branching on vessel by means of anastomosis rings, said instrument being T-shaped, the T/s vertical part being constituted by an unyielding pipe or hose (1) whereas the upper part of the T (3) is elastically expandable and the upper part of the T (3) constituting a closed space but for a connection (2) with the vertical hose or pipe (1).

2. Instrument according to claim 1, characterized in that the upper part of the T (3) is constituted by a thin-walled elastic hose that has been closed at its end (4, 5) and joined to the vertical pipe or hose (1).

3. Instrument according to claim 1 or 2, characterized in that the upper part of the T (3) is about 8 mm long and 2 mm in diameter in unpressurized state.

4. Instrument according to any of the previous claims, characterized in that the vertical hose or pipe (1) is about 1 mm in diameter and thick-walled.

## Patentansprüche

1. Instrument zum Vermeiden von Verengungen im Bereich der Verbindungszone beim Erstellen einer Gefäßverzweigung mit Hilfe von Anastomoseringen, bei dem das Instrument T-förmig ist, der vertikale Teil des T von einem unnachgiebigen Rohr oder Schlauch (1) gebildet wird, während das obere Teil des T (3) elastisch expandierbar ist und das obere Teil des T (3) einen geschlossenen Raum aber für eine Verbindung (2) mit dem vertikalen Schlauch oder dem vertikalen Rohr (1) bildet.

2. Instrument nach Anspruch 1, dadurch gekennzeichnet, daß das obere Teil des T (3) von einem dünnwandigen, elastischen Schlauch gebildet wird, welcher an seinem Ende (4, 5) geschlossen und mit dem vertikalen Rohr oder Schlauch (1) verbunden ist.

3. Instrument nach Anspruch 1 oder 2, dadurch gekennzeichnet, daß das obere Teil des T (3) etwa 8 mm lang und 2 mm im Durchmesser im nicht unter Druck stehenden Zustand bemessen ist.

4. Instrument nach einem der vorangehenden Ansprüche, dadurch gekennzeichnet, daß der vertikale Schlauch oder das Rohr (1) einen Durchmesser von etwa 1 mm hat und dickwandig ist.

**Revendications**

1. Instrument pour empêcher la formation d'étranglements dans la zone de jonction lorsqu'on effectue un branchement sur un vaisseau par des moyens à anneaux d'anastomose, ledit instrument ayant une forme en T, la partie verticale du T étant constituée par un conduit ou tuyau (1) résistant tandis qu'au contraire la partie supérieure du T (3) est extensible élastiquement et la partie supérieure du T (3) constitue un espace fermé sauf à l'endroit d'un raccordement (2) avec le tuyau ou conduit (1) vertical.

2. Instrument selon la revendication 1, caractérisé en ce que la partie supérieure du T (3) est constituée par un tuyau élastique à paroi fine qui a été fermé à ses extrémités (4,5) et relié au conduit ou tuyau (1) vertical.

3. Instrument selon la revendication 1 ou 2, caractérisé en ce que la partie supérieure du T (3) a environ 8 mm de long et 2 mm de diamètre dans un état sans pression.

4. Instrument selon l'une quelconque des revendications précédentes, caractérisé en ce que le tuyau ou le conduit (1) a environ 1 mm de diamètre et a une paroi épaisse.

FIG.1

FIG.3

FIG.2

4

3

5

2

1

EP 0 294 442 B1